# EUROPEAN PATENT APPLICATION

(11) **EP 2 644 603 A1**
(43) Date of publication of application: **02.10.2013**
(21) Application number: 12162491.0
(22) Date of filing: 30.03.2012
(51) Int. Cl.: C07D 295/088, C07C 41/26, C07C 43/23

(54) **Synthetic route for the preparation of substituted 2-phenyl-1,2,3,4-tetrahydronaphthalene-1-ols**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates in general to the field of organic chemistry and in particular to the preparation of 2-phenyl-1,2,3,4-tetrahydronaphthalene-1-ol substituted with a protected hydroxyl group. These compounds can be used as intermediates in the synthesis of pharmaceutically active agents such as lasofoxifene or derivatives thereof.

## Description

### Field of the Invention

The present invention relates in general to the field of organic chemistry and in particular to the preparation of substituted 2-phenyl-1,2,3,4-tetrahydronaphthalene-1-ols. These compounds can be used as intermediates in the synthesis of pharmaceutically active agents such as lasofoxifene or derivatives thereof.

### Background of the Invention

The substituted 2-phenyl-1,2,3,4-tetrahydronaphthalene-1-ols represent useful intermediates for the preparation of active pharmaceutical active agents. For example, lasofoxifene, a non-steroidal selective estrogen receptor modulator (SERM) comprises a 6-hydroxy-2-phenyl-1,2,3,4-tetrahydronaphthalene moiety substituted at its 1-position with a 4-phenoxyethylpyrrolidine moiety.

Selective estrogen receptor modulators (SERMs) are a group of compounds that are classified to act on estrogen receptors. In contrast to pure receptor agonists and antagonists, SERMs reveal different effects depending on the tissue they are applied to. SERMs thereby offer the possibility to be selectively used in various tissues to inhibit or stimulate estrogen-like actions. Further, some SERMs may act as good replacements for hormone replacement therapy (HRT). Lasofoxifene as one selective estrogen receptor modulator was found to be effective for many applications and can be used against osteoporosis, breast cancer and the vaginal atrophy.
D. Hobbs-Mallyon et al., J. Chem. Soc., Perkin Trans. 1, 1997, issue 10, pages 1511-1516 disclose preparation of racemic 6-methoxy-2-phenyl-1,2,3,4-tetrahydronaphthalene-1-ol by reduction of 6-methoxy-2-phenyl-1,2,3,4-tetrahydronaphthalene-1-one using sodium borohydride as reducing reagent.

The object of the present invention is to provide an improved process for preparing substituted 2-phenyl-1,2,3,4-tetrahydronaphthalene-1-ols representing valuable key intermediates for the preparation of pharmaceutically active agents such as lasofoxifene or derivatives thereof.

### Summary of the Invention

Various aspects, advantageous features and preferred embodiments of the present invention as summarized in the following items, respectively alone or in combination, contribute to solving the object of the invention.
(1) A process for preparing a compound of formula II 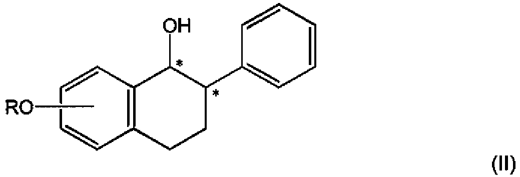 wherein -OR is an ether-type substituent,
   by transfer hydrogenation, in which an organic molecule acts as hydrogen source, of a compound of formula I 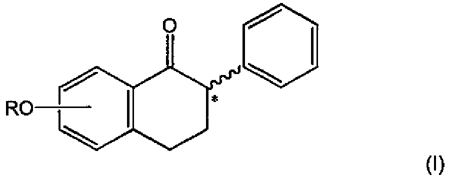 wherein -OR is defined as above,
   wherein transfer hydrogenation is conducted in the presence of a catalyst selected from a complex comprising a transition metal.

The designation "*" in the structural formulae of compounds of formula I and II indicates a chiral center.

The term "chiral center" as used herein means a tetrahedral carbon atom having four different substituents attached thereto. The arrangement of these different substituents around the asymmetric atom determines its optical activity which is determined in the art by the terms S or R and D or L respectively.

The term "transition metal" as used herein means any element in the d-block of the periodic table, which includes groups 3 to 12 of the periodic table.

The term "ether-type substituent" as used herein means that a carbon or silicium atom of the -OR group is adjacent to the oxygen located at the 1,2,3,4-tetrahydronaphthalene ring structure, wherein a C-O-C or Si-O-C ether bond is formed.
(2) The process according to item (1), wherein the R in the ether-type substituent -OR is selected from the group consisting of linear or branched alkyl, aryl, linear or branched alkylaryl, linear or branched alkoxyalkyl, linear or branched alkoxyalkoxyaryl, alkylthioalkyl, alkylaryloxyalkyl, trialkylsilyl, aryldialkylsilyl, diarylalkylsilyl, triarylalkylsilyl, fialkylalkoxyalkyl, preferably R is selected from the group consisting of linear or branched alkyl, linear or branched alkoxyalkyl, aryldialkylsilyl, diarylalkylsilyl and triarylalkylsilyl.

The term "alkyl" as used herein means straight, branched or cyclic hydrocarbons of 1 to 12 carbon atoms, preferably 1 to 8 carbon atoms, more preferably 1 to 6 carbon atoms, and even more preferably 1 to 4 carbon atoms.

The term "aryl" as used herein means hydrocarbon aryls, preferably single or condensed six-membered rings, more preferably phenyl or naphthyl, in particular phenyl.

The term "alkylaryl" as used herein means that the aforementioned aryl moieties are incorporated into the aforementioned straight or branched alkyl moieties either at one of the proximal or distal ends of the alkyl chain or between the aforementioned alkyl chains. For example, proximal end means for ether type substituent -OR adjacent to the oxygen bound to the benzene ring of the 1,2,3,4-tetrahydronaphthalene ring structure of compounds of formulae I and II, while distal means the terminal carbon of the alkyl moiety which is furthermost from said oxygen. Preferably, in the alkylaryl moiety, the aryl moiety is located at the distal end of the alkyl moiety.

The term "substituted" as employed herein means that the aforementioned alkyl, aryl and alkylaryl groups have one, two or three linear or branched C₁-C₆ alkyl or alkoxy substituents.
(3) The process according to item (1) or (2), wherein R in the ether-type substituent -OR is selected from the group consisting of methyl, ethyl, propyl, isopropyl, t-butyl, phenyl, benzyl, methoxymethyl (MOM) and tert-butyldiphenylsilyl (TBDPS), preferably methyl.
(4) The process according to any one of the preceding items, wherein the transition metal of the catalyst complex is selected from the group consisting of ruthenium (Ru), rhodium (Rh) and iridium (Ir), preferably the transition metal is Ru.
(5) The process according to any one of the preceding items, wherein the catalyst has chirality in its ligand(s) and/or at the transition metal atom, or the transition metal complex having chirality is formed *in situ* by using an achiral procatalyst comprising the transition metal together with a cocatalyst having chirality.
(6) The process according to item (5), wherein chiral component(s) is/are in enantiopure or diastereomerically pure form.

The terms "chiral"/"chirality" as used herein means that a compound can not be superimposed on its mirror image.

The terms "enantiopure" and "diastereomerically pure means that an enantiomer or diastereomer is present in a purity of at least 99% enantiomeric excess and 99% diastereomeric excess respectively, preferably in a purity of 99.5-100% enantiomeric excess and 99.5-100% diastereomeric excess respectively. The term "enantiomer" as used herein means two stereoisomers which are mirror images of one another, but which are not superimposable. Enantiomers may also be denoted by the direction into which a solution of the enantiomer rotates the plane of polarization of a beam of polarized light. In case the enantiomer rotates the plane of polarization of a beam of polarized light in a clockwise direction, the enantiomer is denoted as (+)-enantiomer, and in case of counter-clockwise rotation, the enantiomer is denoted as (-)-enantiomer.

The term "diastereomers" as used herein means stereoisomers which do not represent enantiomers.
(7) The process according to any one of the preceding items, wherein the catalyst complex comprises a chiral diamine ligand or a moiety deriving from the chiral diamine ligand.
(8) The process according to item (7), wherein the diamine ligand or moiety deriving from the chiral diamine ligand is a diphenylethyleneamine (DPEN) ligand, more preferably a monotosylated DPEN ligand, even more preferably a tethered monotosylated DPEN ligand, particularly the tethered monotosylated DPEN ligand is selected from the group consisting of (R,R) or (S,S) N-(toluenesulfonyl),N•-(propyl-3-phenyl)-1,2-diphenylenediamine (TsDPEN-teth), (R,R) or (S,S) N-(methanesulfonyl),N•-(propyl-3-phenyl)-1,2-diphenylenediamine (MsDPEN-teth).

The term "tethered" as used herein means that an alkylaryl moiety is covalently bound to the non-sulfonated amine functionality of the DPEN ligand.

The designations "*R"* and "*S*" used herein indicate the absolute configuration at a chiral center(s) of the respective compounds, determined by means of the Cahn Ingold Prelog convention (CIP-convention) known in the art (cf. e.g. M.B. Smith, J. March, "March's Advanced Organic Chemistry: Reactions, Mechanisms and Structure", 6th edition, John Wiley&Sons, Inc., p. 155-158).
(9) The process according to any one of the preceding items, wherein the catalyst complex is selected from the group consisting of [(R,R)-Ms-DPEN-teth RuCl] having the structural formula 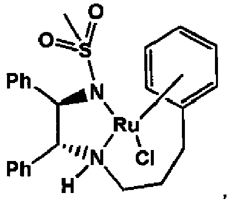 [(R,R)-Ts-DPEN-teth RuCl] having the structural formula 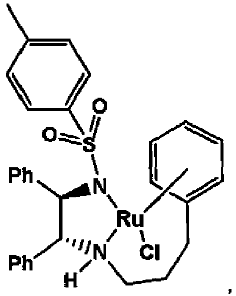 [(S,S)-Ms-DPEN-teth RuCl] having the structural formula 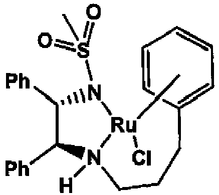 and [(S,S)-TsDPEN-teth RuCl] having the structural formula

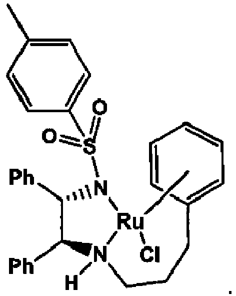

As to the designations "*R*" and "*S*" used in the above compound names, reference is made to the explanations under item (8) above.
(10) The process according to any one of the preceding items, wherein transfer hydrogenation is carried out in a mixture of formic acid and triethylamine.
(11) The process according to item (10), wherein the mixture comprises formic acid and triethylamine in a volume ratio of 2/1 to 3/1, preferably 4.5/2 to 5.5/2.
(12) The process according to any one of the preceding items, wherein the transfer hydrogenation reaction is carried out at a temperature between 25 to 90°C, preferably 30 to 80°C, more preferably 35 to 70°C, even more preferably 36 to 60°C, yet even more preferably 38 to 50°C, and in particular 40 to 45°C.
(13) The process according to any one of the preceding items, wherein the transfer hydrogenation reaction is carried out for a reaction time of 15 to 100 h, preferably 20 to 70 h.
(14) The process according to any one of the preceding items, wherein the molar ratio of substrate to catalyst is at least 20/1, preferably 30/1 to 500/1, more preferably 35/1 to 250/1, even more preferably 40/1 to 150/1, yet even more preferably 75/1 to 125/1, and in particular 90/1 to 110/1.
(15) A process for preparing a compound of formula II 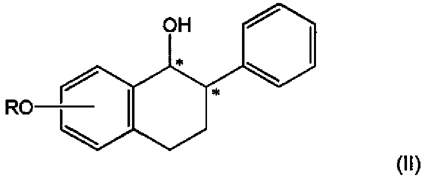 wherein -OR is an ether-type substituent,
   by hydrogenation with elemental hydrogen (H₂) of a compound of formula I 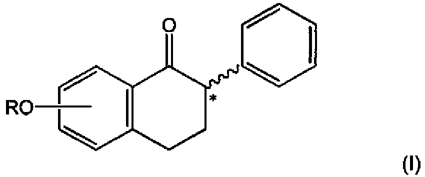 wherein -OR is defined as above,
   wherein hydrogenation is conducted in the presence of a catalyst selected from a complex comprising a transition metal.

As to the designation "*" in the structural formulae of compounds of formula I and II and to terms "transition metal", and "ether-type substituent", reference is made to the explanations under item (1) above.
(16) The process according to item (15), wherein -OR is ether-type substituent as defined in item (2) or (3).
(17) The process according to item (15) or (16), wherein the catalyst is a catalyst as defined in any one of items (2) to (4).
(18) The process according to any one of items (15) to (17), wherein the catalyst complex comprises a chiral diamine ligand or a moiety deriving from the chiral diamine ligand, preferably the diamine ligand or moiety deriving from the chiral diamine ligand is a diphenylethyleneamine (DPEN) ligand, more preferably a monotosylated DPEN ligand, even more preferably the monotosylated DPEN ligand is selected from the group consisting of (R,R) or (S,S) N-(methanesulfonyl)-1,2-diphenylenediamine (MsDPEN), (R,R) or (S,S) N-(toluenesulfonyl)-1,2-diphenylenediamine (TsDPEN), (R,R) or (S,S) N-(toluenesulfonyl),N•-(propyl-3-phenyl)-1,2-diphenylenediamine (TsDPEN-teth), (R,R) or (S,S) N-(methanesulfonyl),N•-(propyl-3-pheny)-1,2-diphenylenediamine (MsDPEN-teth).

As to the designations "*R*" and "*S*" used in the above compound names, reference is made to the explanations under item (8) above.
(19) The process according to any one of items (15) to (18), wherein the catalyst complex is selected from the group consisting of
   [(R,R)-Ms-DPEN RuCl (p-cym)] having the structural formula 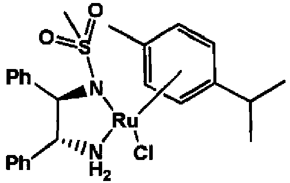 [(R,R)-Ts-DPEN RuCl (p-cym)] having the structural formula 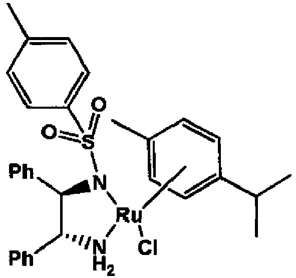 [(R,R)-Ms-DPEN-teth RuCl] having the structural formula 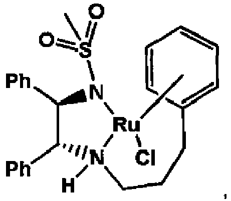 [(R,R)-Ts-DPEN-teth RuCl] having the structural formula 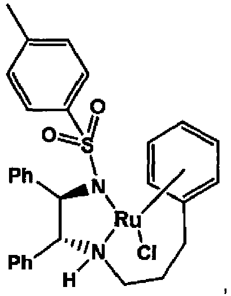 [(R,R)-MsDPEN RuCl (mes)] having the structural formula 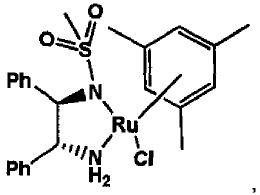 [(S,S)-Ms-DPEN RuCl (p-cym)] having the structural formula 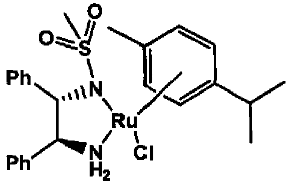 [(S,S)-Ts-DPEN RuCl (p-cym)] having the structural formula 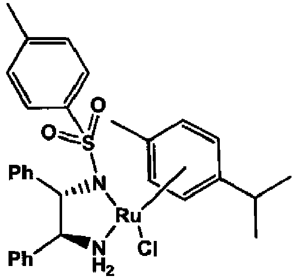 [(S,S)-Ms-DPEN-teth RuCl] having the structural formula 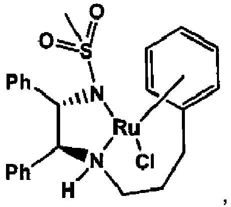 [(S,S)-Ts-DPEN-teth RuCl] having the structural formula 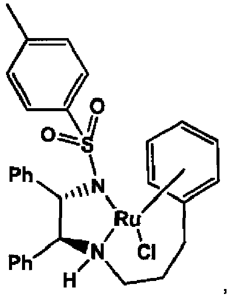 and
   [(S,S)-Ms-DPEN RuCl (mes)] having the structural formula 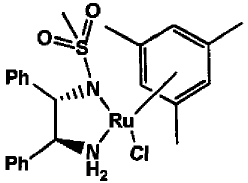 preferably the catalyst complex is selected from the group consisting of [(R,R)-Ms-DPEN-teth RuCl] and [(R,R)-Ms-DPEN RuCl (mes)], more preferably [(R,R)-MsDPEN RuCl (mes)].

As to the designations "*R*" and "*S*" used in the above compound names, reference is made to the explanations under item (8) above.
(20) The process according to any one of items (15) to (19), wherein hydrogenation is carried out in a C1-C4 alcohol as the solvent, preferably methanol.
(21) The process according to any one of items (15) to (20), wherein hydrogenation is carried out in the presence of an aqueous solution comprising an amine compound selected from the group consisting of triethylamine (Et₃N), diisopropyl ethyl amine (iPr₂EtN) and tetramethylguanidine (TMG), preferably Et₃N and TMG, more preferably TMG.
(22) The process according to item (21), wherein the aqueous solution comprises the amine compound in an amount of 10 to 40% by weight relative to the total weight of the aqueous solution, preferably 20 to 30% by weight.
(23) The process according to any one of items (15) to (22), wherein hydrogen applied at a hydrogen pressure of 20 to 40 bar, preferably 25 to 35 bar, more preferable 28 to 32 bar.
(24) The process according to any one of items (15) to (23), wherein the hydrogenation reaction is carried out at a temperature between 30 to 80°C, preferably 35 to 75°C, more preferably 60 to 70°C.
(25) The process according to any one of items (15) to (24), wherein the hydrogenation reaction is carried out for a reaction time of 12 to 100 h, preferably 14 to 85 h, more preferably, in case reaction temperature is between 60 to 70°C, reaction time is 40 to 85 h.
(26) The process according to any one of the preceding items, wherein the molar ratio of substrate to catalyst is at least 20/1, preferably 30/1 to 500/1, more preferably 35/1 to 250/1, even more preferably 40/1 to 150/1, yet even more preferably 75/1 to 125/1, and in particular 90/1 to 110/1.
(27) The process according to any one of the preceding items, wherein compound of formula II is obtained in a diastereomeric ratio of *cis*-isomer to trans-isomer of at least 100/1 and an enantiomeric excess of the *cis*-configured compound of at least 90%, more preferably enantiomeric excess of the *cis*-configured compound is at least 94%, even more preferably at least 96%, yet even more preferably at least 98%, and in particular at least 99%.

The term "diastereomeric ratio" as used herein means the molar ratio of cis-isomers to *trans*-isomers of compound of formula II. In this context, the term "*cis*-isomer" means that the OH substituent at 1-position and the phenyl substituent at 2-position of compound of formula II are located on the same side with respect to the cyclopropane ring plane, which is indicated by bonds depicted by two bold (that is, both substituents are located above the cyclopropane ring plane) or two dashed lines (that is, both substituents are located below the cyclopropane ring plane):

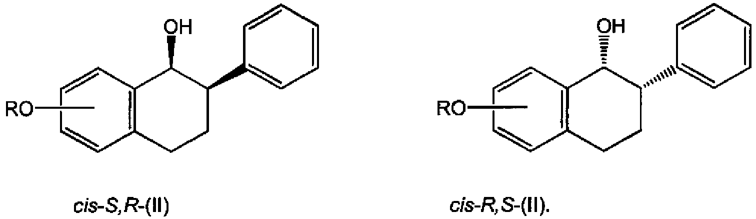

The term "*trans*-isomer" means that the OH substituent at 1-position and the phenyl substituent at 2-position of compound of formula II are located on opposite sides with respect to the cyclopropane ring plane, which is indicated by bonds depicted by one bold and one dashed line respectively:

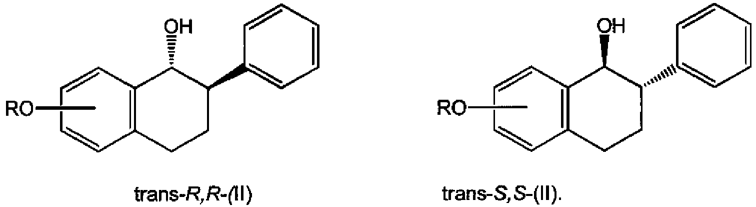

The term "enantiomeric excess" as used herein means the difference between the percentage of one enantiomer of an optically active compound and the percentage of the other enantiomer of the same optically active compound. For example, an optically active compound which contains 75% R-enantiomer and 25% S-enantiomer will have an enantiomeric excess of 50% of R-enantiomer.
(28) A process for preparing a compound of formula X 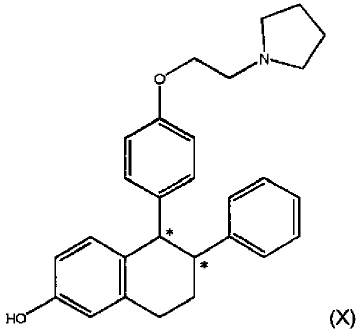 or a pharmaceutical acceptable salt thereof, the process comprising:
   a) preparing a compound of formula II, 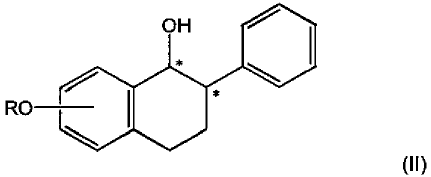 wherein -OR is an ether-type substituent,
      by a process according to any one of items (1) to (27), and
   b) further converting the compound of formula II to a compound of formula X
      or a pharmaceutically acceptable salt thereof.
   As to the designation "*", reference is made to the explanations under item (1) above.
(29) A process according to item (28), wherein compound of formula X is lasofoxifene (X') having the structural formula 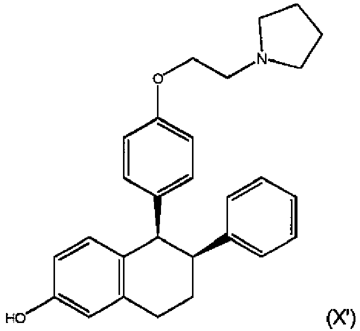 in which the substituents at 1- and 2-position are in (1*R*,2*S*)-configuration.

As to the designations "*R*" and "*S*" used in connection with the stereochemistry at 1- and 2-position of compound of formula (X'), reference is made to the explanations under item (8) above.
(30) A process for the preparation of a pharmaceutical composition comprising a compound of formula X or a pharmaceutically acceptable salt thereof as active ingredient, comprising the steps of:
   a) preparing a compound of formula X or a salt thereof according to the process according to item (28) or (29), and
   b) admixing the prepared compound of formula X or pharmaceutically acceptable salt thereof with at least one pharmaceutically acceptable excipient.

### Detailed description of the invention

The present invention is now described in more detail by referring to further preferred and further advantageous embodiments and examples, which are however presented for illustrative purposes only and shall not be understood as limiting the scope of the present invention.

Conventionally, chiral 2-phenyl-1,2,3,4-tetrahydronaphthalene-1-ols substituted with a protected hydroxyl group are synthesized by means of chemical synthesis affording the desired 1,2,3,4-tetrahydronaphthalene derivative in the form of a racemate. Therefore, in order to obtain enantiomerically enriched or pure 2-phenyl-1,2,3,4-tetrahydronaphthalene-1-ols substituted with a protected hydroxyl group, in conventional processes, it is necessary to subject the racemic compound to resolution of racemates. Resolution of racemates is a laborious procedure, since it typically requires multiple subsequent resolution steps in order to obtain a satisfactory high degree of enantio-enrichment. These multiple resolution steps in turn result in relatively poor yields of enantiomerically enriched or enantiopure product.

P. Peach et al., Tetrahedron, 2006, 62, pages 1864 to 1876 disclose transfer hydrogenation of • - substituted ketones involving dynamic kinetic resolution wherein among others, 2-phenyl-1,2,3,4-tetrahydronaphthaiene-1(2H)-one is converted to *cis*-(-)-2-phenyl-1,2,3,4-tetrahydronaphthalene-1-ol having a diasteroemeric ratio of *cis*/*trans*-isomer higher than 97/1, and an enantiomeric excess of at least 98%:

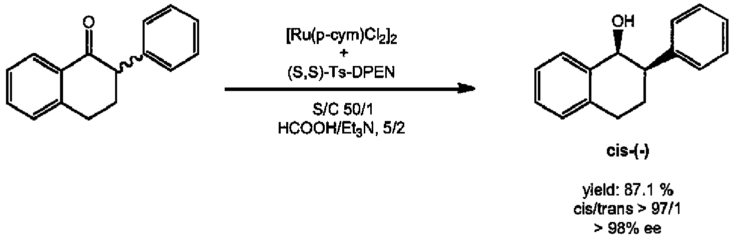

In this document, a relatively simple 1,2,3,4-tetrahydronaphthalene substrate is used which is not substituted at the aromatic phenyl moiety. However, distinct from such a relatively simple substrate compound for pharmaceutical active agents substitution of any one of the position 5,6,7 or 8 of the aromatic phenyl ring is useful or even necessary. For example, in a non-steroidal selective estrogen receptor modulator (SERM) such as lasofoxifene, a hydroxy group is present in the position 6, as can be gathered from the below structural formula in which the positions of the 1,2,3,4-tetrahydronaphthalene ring structure of lasofoxifene are indicated:

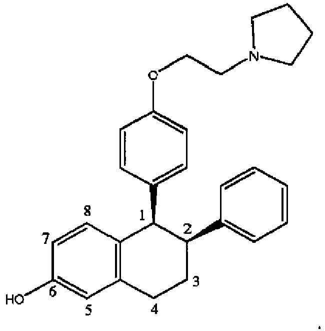

Therefore, 2-phenyf-1,2,3,4-tetrahydronaphthalene-1-ols being substituted with a protected hydroxyl group at any one of positions 5, 6, 7 and 8 of the 1,2,3,4-tetrahydronaphthalene ring structure represent valuable intermediate compounds in the field of pharmacy and have been surprisingly found, albeit their further substituent, to be susceptible substrates for transfer hydrogenation or H₂-hydrogenation applying complexes comprising a transition metal. Even further surprisingly, 2-phenyl-1,2,3,4-tetrahydronaphthalene-1-ol being substituted with a protected hydroxyl group at any one of positions 5, 6, 7 and 8 can be obtained in advantageous high enantiomeric excess combined with beneficial properties, such as high diastereomeric excess and/or conversion/yield.

In particular, it was surprisingly found by the present inventors that a 1,2,3,4-tetrahydronaphthalene derivative having the formula I 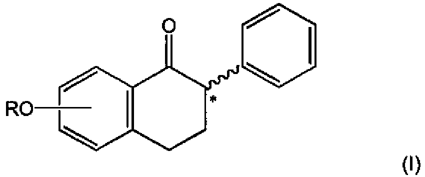 wherein -OR is an ether-type substituent ,
represents a particularly suitable substrate for both H₂-hydrogenation and transfer hydrogenation reactions. Furthermore, especially when selecting particular types of transition metal complexes, it was possible to obtain both (-)- and (+)-enantiomer of compound of formula II in an advantageous high enantiomeric excess or even in enantiomerically pure state.

The compound of formula I can be prepared by organic synthesis in order to be provided, preferably compound of formula I is provided in racemic form. For example, a compound of formula I in which R in the ether-type substituent -OR is methyl can be prepared by a metal catalyzed coupling reaction illustrated in Scheme 1 below.

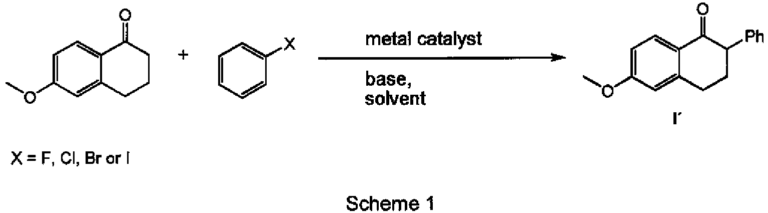

Analogous reactions can be carried out with corresponding substituents for the "OR" group instead of the methyl group shown in Scheme 1 above.

According to one aspect of the invention, a compound of formula II 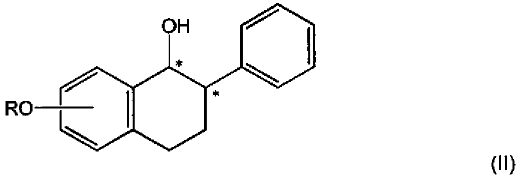 wherein -OR is an ether-type substituent,
is prepared by a process comprising transfer hydrogenation, in which an organic molecule acts as hydrogen source, of a compound of formula I 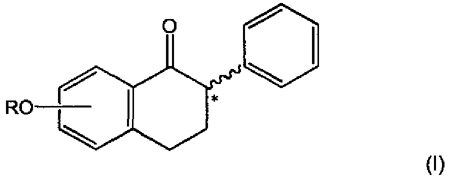 wherein -OR is defined as above,
wherein transfer hydrogenation is conducted in the presence of a catalyst selected from a complex comprising a transition metal.

Wherein the compound of formula II is the starting material for a target molecule or an intermediate comprising the hydroxy group (-OR, wherein R is H), the substituent R in the ether-type substituent "-OR" can be selected from any hydroxy protecting group known in the art. Preferably, "-OR" is substantially inert to hydrogenation conditions and the cleavage conditions for removing said hydroxy protecting group will not adversely affect the structure of compound of formula II or a derivative thereof.

The ether-type substituent-OR located at the phenyl ring of 2-phenyl-1,2,3,4-tetrahydronaphthalene-1-ol moiety of compounds of formulae I and II can be located at any one of positions 5, 6, 7 or 8 of the 1,2,3,4-tetrahydronaphthalene ring structure. Preferably, -OR group is located at the position 6 of the 1,2,3,4-tetrahydronaphthalene ring structure, as exemplary illustrated below for compound of formula II

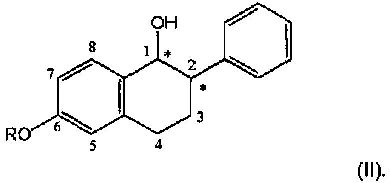

The procedural concept according to this aspect of the invention provides for unexpectedly high, preferably almost quantitative yield owing to dynamic kinetic resolution (DKR) of the starting material of compound of formula I. The term "dynamic kinetic resolution" (DKR) as used herein means that a starting material can racemise under the reaction conditions, wherein the faster reacting enantiomer is continually removed from the system by an irreversible reaction. Hence, DKR provides for remarkably high, even up to 100% yield, given that racemisation of the starting material is much faster than the irreversible reaction. In the present process, the starting material in the form of compound of formula I is a ketone which racemises due to keto-enol tautomerism.

Furthermore, catalytic transfer hydrogenation provides for a significantly simplified purification compared to purification of compounds of formula II obtained by means of conventional reduction of a keto group by a hydride reagent such as NaBH₄. Compound of formula II represents a highly valuable intermediate compound for the preparation of pharmaceutically active agents such as lasofoxifene or derivatives thereof. Instead of quite different synthetic schemes and instead of other many possible strategies to convert the keto- into the hydroxyl group by other hydrogenation or other reduction modalities, transfer hydrogenation has been found to be particularly effective when applied to the compound of formula I.

Importantly, the protected hydroxyl group located at any one of the positions 5, 6, 7 or 8 of the 1,2,3,4-tetrahydronaphthalene ring does not affect, but surprisingly still provides for fast keto-enol tautomerism combined with effective transfer hydrogenation susceptible to DKR.

According to a preferred embodiment, R in the ether-type substituent -OR is selected from the group consisting of linear or branched alkyl, aryl, linear or branched alkylaryl, linear or branched alkoxyalkyl, linear or branched alkoxyalkoxyaryl, alkylthioalkyl, alkylaryloxyalkyl, trialkylsilyl, aryldialkylsilyl, diarylalkylsilyl, triarylalkylsilyl, trialkylalkoxyalkyl, preferably R is selected from the group consisting of linear or branched alkyl, linear or branched alkoxyalkyl, aryldialkylsilyl, diarylalkylsilyl and triarylalkylsilyl. More preferably, the R is selected from the group consisting of methyl, methoxymethyl (MOM) and *tert*-butyldiphenylsilyl (TBDPS), and in particular, R is methyl.

In this way, the ether-type substituent -OR is suitably selected with the proviso that both -OR is substantially inert to hydrogenation conditions, in particular to transfer hydrogenation conditions, and that the cleavage conditions for removing -OR will not adversely affect the structure of compound of formula II or a derivative thereof.

According to another preferred embodiment, the transition metal of the catalyst complex is selected from the group consisting of ruthenium (Ru), rhodium (Rh) and iridium (Ir), preferably the transition metal is Ru.

In a further preferred embodiment, the catalyst complex has chirality in its ligand(s) and/or at the transition metal atom, or the transition metal complex having chirality is formed *in situ* by using an achiral procatalyst comprising the transition metal together with a cocatalyst having chirality. Preferably, chiral component(s) of the catalyst complex is/are in enantiopure or diastereomerically pure form.

Suitable chiral component(s) for the catalyst complex are for example chiral diamine ligand(s) or a moiety deriving from the chiral diamine ligand. Preferably, the diamine ligand or moiety deriving from the chiral diamine ligand comprised in the catalyst complex is a diphenylethyleneamine (DPEN) ligand, more preferably a monotosylated DPEN ligand, even more preferably a tethered monotosylated DPEN ligand.

According to a preferred embodiment, the catalyst complex comprises a tethered monotosylated DPEN ligand selected from the group consisting of (R,R) or (S,S) N-(toluenesulfonyl),N•-(propyl-3-phenyl)-1,2-diphenylenediamine (TsDPEN-teth), (R,R) or (S,S) N-(methanesulfonyl),N•-(propyl-3-phenyl)-1,2-diphenylenediamine (MsDPEN-teth).

According to a particularly preferred embodiment, the catalyst complex is selected from the group consisting of
[(R,R)-Ms-DPEN-teth RuCl] having the structural formula 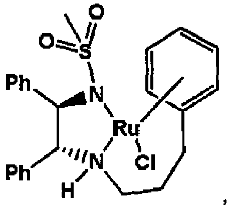 [(R,R)-Ts-DPEN-teth RuCl] having the structural formula 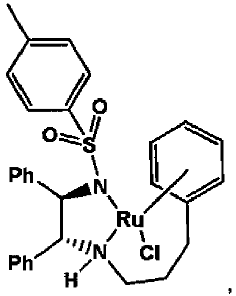 [(S,S)-Ms-DPEN-teth RuCl] having the structural formula 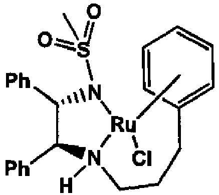 and [(S,S)-TsDPEN-teth RuCl] having the structural formula

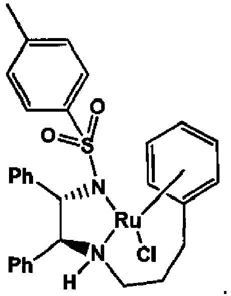

According to the above embodiments relating to specific selections of components of the catalyst complex or a specific selection of the catalyst complex, a compound of formula II having optical activity can be obtained, which optical activity can be beneficially controlled by selecting particularly suitable metals and/or ligands for the catalyst complex, or by selecting particularly suitable catalyst complexes. In particular, it was surprisingly found that complex catalysts comprising diamine ligands, preferably DPEN ligands provide for surprising inductions of high diastereomeric ratio and enantiomeric excess in compound of formula II.

According to a further preferred embodiment, transfer hydrogenation is carried out in a mixture of formic acid and triethylamine.

In this way, a particularly suitable hydrogen source in form of formic acid is selected, wherein triethylamine provides for irreversibility of the transfer hydrogenation reaction.

In a further preferred embodiment, in the transfer hydrogenation process, the reaction mixture comprises formic acid and triethylamine in a volume ratio of 2/1 to 3/1, preferably 4.5/2 to 5.5/2.

Preferably, the transfer hydrogenation reaction is carried out at a temperature between 25 to 90°C, more preferably 30 to 80°C, even more preferably 35 to 70°C, yet even more preferably 36 to 60°C, still yet even more preferably 38 to 50°C, and in particular 40 to 45°C.

Suitable reaction times for the transfer hydrogenation reaction according to the present invention are 15 to 100 h, preferably transfer hydrogenation is carried out for 20 to 70 h.

As regards the molar ratio of substrate to catalyst in the present transfer hydrogenation, such ratio is preferably at least 20/1, more preferably 30/1 to 500/1, even more preferably 35/1 to 250/1, yet even more preferably 40/1 to 150/1, still yet even more preferably 75/1 to 125/1. and in particular 90/1 to 110/1.

According to another aspect of the invention, a compound of formula II 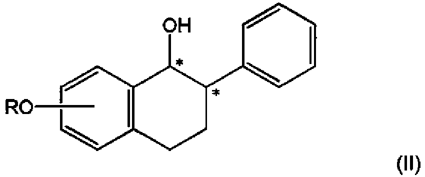 wherein -OR is an ether-type substituent.
is prepared by a process comprising hydrogenation with elemental hydrogen (H₂) of a compound of formula I 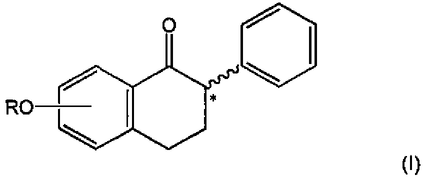 wherein -OR is defined as above,
wherein hydrogenation is conducted in the presence of a catalyst selected from a complex comprising a transition metal.

According to this aspect of the invention, compound of the formula II can not only be readily prepared from compound of the formula I by catalytic hydrogenation, but also in unexpected high, preferably almost quantitative yield owing to dynamic kinetic resolution (DKR) of the starting material of compound of formula I. Furthermore, catalytic hydrogenation provides for a significantly simplified purification compared to purification of compounds of formula II obtained by means of conventional reduction of a keto group by a hydride reagent such as NaBH₄. Compound of formula II represents a highly valuable intermediate compound for the preparation of pharmaceutically active agents such as lasofoxifene or derivatives thereof.

Compound of formula II represents a highly valuable intermediate compound for the preparation of pharmaceutically active agents such as lasofoxifene or derivatives thereof. Instead of quite different synthetic schemes and instead of other many possible strategies to convert the keto- into the hydroxy group by other hydrogenation or other reduction modalities, hydrogenation has been found to be particularly effective when applied to the compound of formula I.

Surprisingly, the -OR substituent located at the 1,2,3,4-tetrahydronaphthalene ring does not affect, but still provides for fast keto-enol tautomerism combined with effective transfer hydrogenation susceptible to DKR.

According to a preferred embodiment, the protecting group -OR is as defined above for the transfer hydrogenation process.

According to a further preferred embodiment, features (a) to (e) representing particularly suitable selections for components of the catalyst complex for transfer hydrogenation also represent particularly suitable catalyst features for hydrogenation with elemental hydrogen.

According to a preferred embodiment, the catalyst complex comprises a monotosylated DPEN ligand which is selected from the group consisting of (R,R) or (S,S) N-(methanesulfonyl)-1,2-diphenylenediamine (MsDPEN), (R,R) or (S,S) N-(toluenesulfonyl)-1,2-diphenylenediamine (TsDPEN), (R,R) or (S,S) N-(toluenesulfonyl),N•-(propyl-3-phenyl)-1,2-diphenylenediamine (TsDPEN-teth), (R,R) or (S,S) N-(methanesulfonyl),N•-(propyl-3-phenyl)-1,2-diphenylenediamine (MsDPEN-teth).

According to a particularly preferred embodiment,
the catalyst complex is selected from the group consisting of
[(R,R)-Ms-DPEN RuCl (p-cym)] having the structural formula 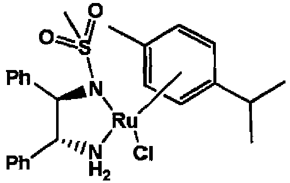 [(R,R)-Ts-DPEN RuCl (p-cym)] having the structural formula 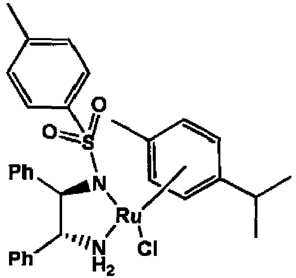 [(R,R)-Ms-DPEN-teth RuCl] having the structural formula 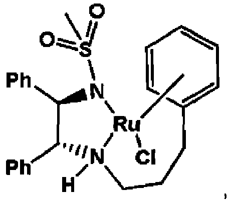 [(R,R)-Ts-DPEN-teth RuCl] having the structural formula 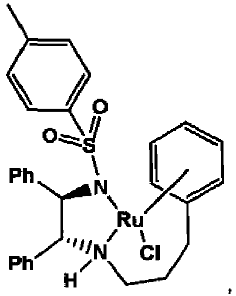 [(R,R)-MsDPEN RuCl (mes)] having the structural formula 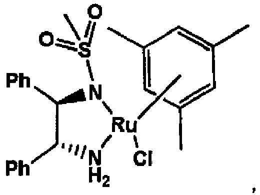 [(S,S)-Ms-DPEN RuCl (p-cym)] having the structural formula 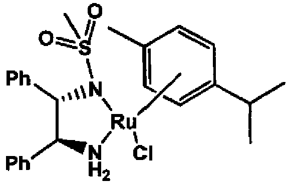 [(S,S)-Ts-DPEN RuCl (p-cym)] having the structural formula 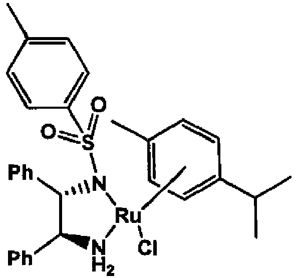 [(S,S)-Ms-DPEN-teth RuCl] having the structural formula 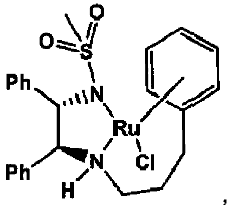 [(S,S)-Ts-DPEN-teth RuCl] having the structural formula 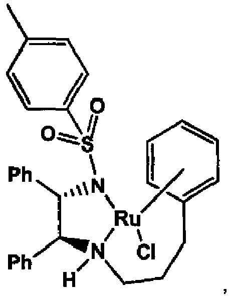 and
[(S,S)-Ms-DPEN RuCl (mes)] having the structural formula 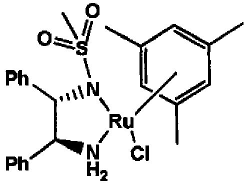 preferably the catalyst complex is selected from the group consisting of [(R,R)-Ms-DPEN-teth RuCl] and [(R,R)-Ms-DPEN RuCl (mes)], more preferably [(R,R)-MsDPEN RuCl (mes)].

According to the above embodiments relating to specific selections of components of the catalyst complex or a specific selection of the catalyst complex, a compound of formula II having optical activity can be obtained, which optical activity can be beneficially controlled by selecting suitable metals and/or ligands for the catalyst complex, or by selecting particularly suitable catalyst complexes. In particular, it was surprisingly found that complex catalysts comprising diamine ligands, preferably DPEN ligands provide for surprisingly induce high diastereomeric ratio and enantiomeric excess in compound of formula II.

According to another preferred embodiment, hydrogenation is carried out in the presence of an aqueous solution comprising an amine compound selected from the group consisting of triethylamine (Et₃N), diisopropyl ethyl amine (iPr₂EtN) and tetramethylguanidine (TMG), preferably Et₃N and TMG, more preferably TMG. Preferably, said aqueous solution comprises the amine compound in an amount of 10 to 40% by weight relative to the total weight of the aqueous solution, preferably 20 to 30% by weight.

According to this preferred embodiment of the invention, it was surprisingly found that by choosing a particularly suitable amine compound, the yield of compound of formula II can be substantially increased, while compound of formula II is obtained in both a desirable diasteriomeric *cis*/*trans* ratio and high enantiomeric excess.

In a further preferred embodiment of the present invention, H₂-hydrogenation is carried out in a C1-C4 alcohol as the solvent, preferably methanol.

Preferably, in the H₂-hydrogenation process according to the present invention, hydrogen is applied at a hydrogen pressure of 20 to 40 bar, preferably 25 to 35 bar, more preferable 28 to 32 bar.

Suitable reaction temperature for the present H₂-hydrogenation lies between 30 to 80°C, preferably 35 to 75°C, more preferably 60 to 70°C.

According to a preferred embodiment, H₂-hydrogenation reaction is carried out for a reaction time of 12 to 100 h, preferably 14 to 85 h, more preferably, in case reaction temperature is between 60 to 70°C, reaction time is 40 to 85 h.

As regards the molar ratio of substrate to catalyst in the present transfer hydrogenation, such ratio is preferably at least 20/1, more preferably 30/1 to 500/1, even more preferably 35/1 to 250/1, yet even more preferably 40/1 to 150/1, still yet even more preferably 75/1 to 125/1, and in particular 90/1 to 110/1.

In both above discussed procedural aspects according to the invention, that is in transfer hydrogenation process and in H₂-hydrogenation process, according to a preferred embodiment, compound of formula II is obtained in a diastereomeric ratio of *cis*-isomer to trans-isomer of at least 100/1 and an enantiomeric excess of the cis-configured compound of at least 90%, more preferably enantiomeric excess of the *cis*-configured compound is at least 94%, even more preferably at least 96%, yet even more preferably at least 98%, and in particular at least 99%. According to this aspect of the invention, compound of formula II is obtained in advantageous to exceptionally high diastereomeric ratio and enantiomeric excess.

According to another aspect of the invention a compound of formula X 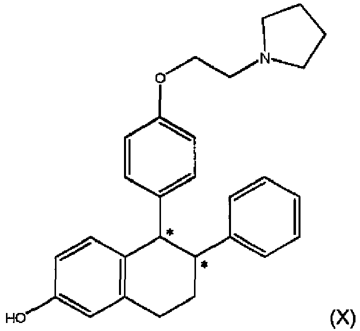 or a pharmaceutical acceptable salt thereof, is provided by a process comprising:
a) preparing a compound of formula II 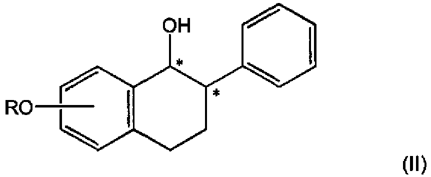 wherein -OR is an ether-type substituent,
   by a process according to any one of items (1) to (26), and
b) further converting the compound of formula II to compound of formula X or a pharmaceutically acceptable salt thereof.

This aspect of the invention provides a particularly advantageous process for preparing lasofoxifene owing to the application of the present procedural aspects of transfer hydrogenation or H₂-hydrogenation and the beneficial effects involved therewith.

According to a preferred embodiment of the invention, in the aforementioned process, compound of formula X is lasofoxifene (X') having the structural formula 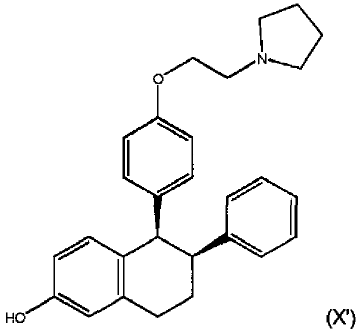 in which the substituents at 1- and 2-position are in (1*R*,2*S*)-configuration.

According to a further aspect of the invention, a pharmaceutical composition comprising compound of formula X or a pharmaceutically acceptable salt thereof as active ingredient, is provided by a process comprising the steps of:
a) preparing compound of formula X or a salt thereof according to the process according to item (27) or (28), and
b) admixing the prepared compound of formula X or pharmaceutically acceptable salt thereof with at least one pharmaceutically acceptable excipient.

The term "pharmaceutically acceptable excipient" as used herein means any physiologically inert, pharmacologically inactive material known in the art being compatible with the physical and chemical characteristics of the active agent. Preferably, the pharmaceutically acceptable excipient is selected from the group consisting of binders, disintegrants, bulk polymers and preservatives.

The term "binder" as used herein means a binding agent which improves adhesion in between particles of the pharmaceutically active agent.

The term "disintegrant" as used herein means an agent providing for rapid disintegration of a pharmaceutical composition into smaller fragments when in contact with water, wherein dissolution of the pharmaceutical composition and in particular of a pharmaceutically active agent comprised therein is improved.

The term "bulk polymer" as used herein means a polymeric filling agent which is typically added to a pharmaceutical composition in large amounts, at least in an amount larger than 6% by weight relative to the total weight of the pharmaceutical composition.

The term "preservatives" as used herein means a substance or mixture of substances which prevents decomposition of a pharmaceutical composition, e.g. microbial or bacterial decomposition.

The following examples are merely illustrative of the present invention and they should not be considered as limiting the scope of the invention in any way. The examples and modifications or other equivalents thereof will become apparent to those versed in the art in the light of the present entire disclosure.

### Examples

### Preparation of compound of formula I'

Compound of formula I' (6-methoxy-2-phenyl-3,4-dihydronaphthalene-1(2H)-one) was prepared as follows: 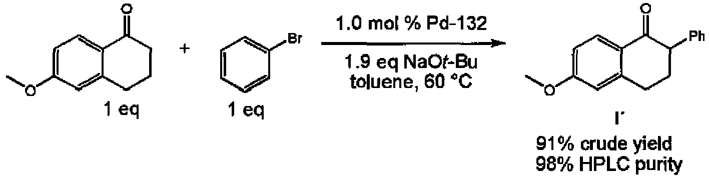 6-Methoxy-3,4-dihydronaphthalene-1(2H)-one (l, 352 mg, 2.0 mmol), NaOt-Bu (365 mg, 3.8 mmol) and Pd-132 (14 mg, 0.02 mmol) were added to a Schlenk flask. The flask was sealed with a rubber stopper, evacuated and backfilled with nitrogen three times. Bromobenzene (0.21 mL, 2.0 mmol) followed by toluene (2.0 mL), was added via airtight syringe through the rubber stopper, and the reaction mixture was stirred at 60 °C for 18 hours. The reaction mixture was filtered on silica gel and the silica pad was washed with MTBE (20 mL). The filtrate was concentrated to give the desired product as an off-white solid (461 mg, 91 %).
¹H NMR (CDCl₃) *f* = 2.39 - 2.44 (m, 2H), 3.0 - 3.1 (m, 2H), 3.8 (t, 1 H), 3.9 (s, 3H), 6.7 (d, 1H), 6.9 (dd, 1H), 7.2 (m, 2H), 7.3 (m, 1H), 7.4 (m, 2H), 8.1 (d, 1H) ppm.
¹³C NMR (CDCl₃) *f* = 28.9, 31.2, 54.0, 55.4, 112.4, 113.2, 126.4, 126.7, 128.35, 128.4, 130.2, 140.0, 146.5, 163.5, 196.9 ppm.

### Example 1: Transfer hydrogenation of compound of formula I'

Compound of formula I' (250 mg, 1 mmol) and the respective amount of catalyst indicated in Table 1 below (e.g. for entries 1 to 4 with substrate/catalyst (S/C) ratio of 50/1: 2 mol% relative to the molar amount of substrate; for entries 5, 6, 8 and 10 with S/C ratio of 100/1: 1 mol%) were placed in a tube under argon atmosphere and a mixture of HCOOH/NEt₃ (3 mL) in a volume ratio of 5/2 was added. The resulting reaction mixture was heated at a temperature of 40 to 45 °C for the time indicated in Table 1 below. After cooling to room temperature, the reaction mixture was quenched with saturated aqueous solution NaHCO₃/AcOEt. The organic phase was diluted in heptane/EtOH and analysed by HPLC to determine conversion to compound of formula II' and to determine diastereomeric ratio (dr) cis/trans and an enantiomeric excess (ee) of compound of formula II' as indicated in Table 1 below.

**Table 1. T = 40-45°C**

| Entry | Catalyst | S/C | Time / h | Conv to II' / % | dr cis/trans | ee cis / % |
|---|---|---|---|---|---|---|
| 1 | [(R,R)-TsDPEN RuCl (p-cym)] | 50/1 | 24 | 5 | >100/1 | - |
| 2 | [(R,R)-TsDPEN teth Ru Cl] | 50/1 | 24 | 88 | >100/1 | >99 (+) |
| 3 | [(S,S)-TsDPEN teth Ru Cl] | 50/1 | 24 | 73 | >100/1 | 99 (-) |
| 4 | [(S,S)-MsDPEN teth Ru Cl] | 50/1 | 24 | 43 | >100/1 | 98 (-) |
| 5 | [(R,R)-MsDPEN Ru Cl (mes)] | 100/1 | 40 | 12 | >100/1 | - |
| 6 | [(R,R)-MsDPEN Ru Cl (mes)] | 100/1 | 64 | 13 | >100/1 | - |
| 7 | [(R,R)-MsDPEN Ru Cl (mes)] | 250/1 | 64 | <1 | >100/1 | - |
| 8 | [(R,R)-MsDPEN teth Ru Cl] | 100/1 | 40 | 88 | >100/1 | >99 (+) |
| 9 | [(R,R)-MsDPEN teth Ru Cl] | 100/1 | 64 | 91 | >100/1 | >99 (+) |
| 10 | [(S,S)-TsDPEN teth Ru Cl] | 100/1 | 64 | 90 | >100/1 | 99 (-) |

The structural formulae of the catalysts of entries 2 to 4 and 8 to 10 of Table 1 are illustrated in item (9) of the summary of invention.

The structural formulae of the catalysts of entries 1 and 5 to 7 of Table 1 are as follows:

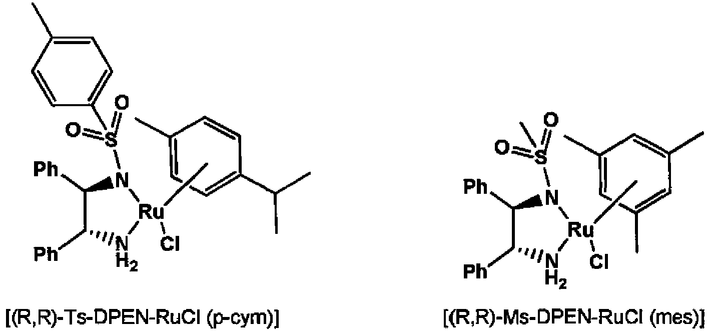

### Example 2: H₂-Hydrogenation of compound of formula I'

Compound of formula I' (250 mg, 1 mmol) and the respective amount of catalyst indicated in Tables 2 and 3 below (e.g. for entries 1 to 5 of Table 3 with substrate/catalyst (S/C) ratio of 100/1: 1 mol% relative to the molar amount of substrate) were weighted in a glass tube that was placed in a Biotage Endeavour catalytic screening system and purged with nitrogen. A stock solution of TMG 25 % was added, followed by methanol as solvent. The reaction was purged three times with hydrogen, placed under hydrogen (3 bar), heated to the respective temperature indicated in Table 2 and 3 below, and pressurized to the respective hydrogen pressure indicated in Tables 2 and 3 below. After the time indicated in Tables 2 and 3 below, the reaction was vented, passed through a pad of silica gel and evaporated to dryness.

**Table 2. Solvent = MeOH, p (H₂) = 30 bar**

| Entry | Catalyst | S/C | T / °C | p(H₂) / bar | Time / h | Conv. to II' / % | dr cis/trans | ee cis / % |
|---|---|---|---|---|---|---|---|---|
| 1 | [(R,R)-MsDPEN teth RuCl] | 100/1 | 50 | 30 | 16 | 38 | >100/1 | >97% (+) |
| 2 | [(S,S)-MsDPEN teth Ru Cl] | 100/1 | 50 | 30 | 16 | 19 | >100/1 | 96(-) |
| 3 | [(R,R)-TsDPEN teth Ru Cl] | 100/1 | 50 | 30 | 16 | 9 | >100/1 | > 97 (+) |
| 4 | [(R,R)-TsDPEN Ru Cl (p-cym)] | 100/1 | 50 | 30 | 16 | 23 | >100/1 | >9 7 (+) |
| 5 | [(S,S)-MsDPEN Ru Cl (p-cym)] | 100/1 | 50 | 30 | 16 | 18 | >100/1 | 95(-) |
| 6 | [R,R-MsDPEN Ir Cp*OTf] | 100/1 | 50 | 30 | 16 | 5 | >100/1 | - |
| 7 | [(R,R)-MsDPEN teth Ru Cl] | 100/1 | 40 | 30 | 64 | 48 | 95/1 | 97 (+) |
| 8 | [(R)- XylPPhosRuCl₂(R)-DAIPEN] | 50/1 | 50 | 20 | 12 | 72 | 1/1.1 | 47 (-) |
| 9 | [(S)- XylPPhosRuCl₂(R)-DAIPEN] | 50/1 | 50 | 20 | 12 | 72 | 1.4/1 | 77 (+) |

The structural formulae of the catalysts of entries 1 to 5 and 7 of Table 2 are illustrated in item (18) of the summary of invention.

The structural formulae of the catalyst of entry 6 of Table 2 and ligands of catalysts of entries 8 and 9 of Table 2 are as follows:

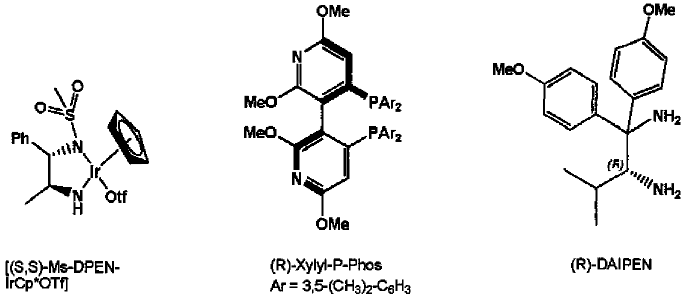

**Table 3. Solvent = MeOH, S/C = 100/1, T = 65 °C, p(H₂) = 30 bar**

| Entry | Catalyst | Base | Time / h | Conv to II' / % | dr cis/trans | ee cis / % |
|---|---|---|---|---|---|---|
| 1 | [(R,R)-MsDPEN teth RuCl] | iPr₂EtN 25 % | 16 | 53 | >53/1 | 92% (+) |
| 2 | [(R,R)-MsDPEN teth Ru Cl] | Et₃N 25 % | 16 | 51 | >100/1 | 93 (+) |
| 3 | [(R,R)-MsDPEN teth Ru Cl] | TMG 25 % | 16 | 39 | 39/1 | 95 (+) |
| 4 | [(R,R)-MsDPEN Ru Cl (mes)] | TMG 25 % | 16 | 44 | >100/1 | 95 (+) |
| 5 | [(R,R)-MsDPEN Ru Cl (mes)] | TMG 25 % | 64 | 80 | >100/1 | 92 (+) |

The structural formulae of the catalysts of entries 1 to 5 of Table 3 are illustrated in item (18) of the summary of invention.

## Claims

1. A process for preparing a compound of formula II 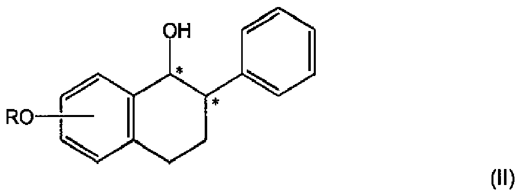 wherein -OR is an ether-type substituent,
by transfer hydrogenation, in which an organic molecule acts as hydrogen source, of a compound of formula I 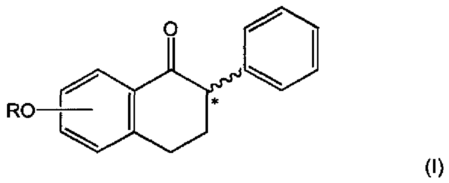 wherein -OR is defined as above,
wherein transfer hydrogenation is conducted in the presence of a catalyst selected from a complex comprising a transition metal.

2. The process according to claim 1, wherein R in the ether-type substituent -OR is selected from the group consisting of linear or branched alkyl, aryl, linear or branched alkylaryl, linear or branched alkoxyalkyl, linear or branched alkoxyalkoxyaryl, alkylthioalkyl, alkylaryloxyalkyl, trialkylsilyl, aryldialkylsilyl, diarylalkylsilyl, triarylalkylsilyl, trialkylalkoxyalkyl, preferably R is selected from the group consisting of linear or branched alkyl, linear or branched alkoxyalkyl, aryldialkylsilyl, diarylalkylsilyl and triarylalkylsilyl, more preferably R is selected from the group consisting of methyl, methoxymethyl (MOM) and tert-butyldiphenylsilyl (TBDPS), even more preferably R is methyl.

3. The process according to any one of the preceding claims, **characterized by** either one or a combination of the following procedural features (a) to (e):
(a) the transition metal of the catalyst complex is selected from the group consisting of ruthenium (Ru), rhodium (Rh) and iridium (Ir), preferably the transition metal is Ru;
(b) the catalyst complex has chirality in its ligand(s) and/or at the transition metal atom, or the transition metal complex having chirality is formed *in situ* by using an achiral procatalyst comprising the transition metal together with a cocatalyst having chirality;
(c) chiral component(s) of the catalyst complex is/are in enantiopure or diasteriomerically pure form;
(d) the chiral component(s) are chiral diamine ligand(s) or a moiety deriving from the chiral diamine ligand;
(e) a diamine ligand or moiety deriving from the chiral diamine ligand comprised in the catalyst complex is a diphenylethyleneamine (DPEN) ligand, more preferably a monotosylated DPEN ligand.

4. The process according to any one of the preceding claims, wherein the catalyst complex comprises a tethered monotosylated DPEN ligand, preferably the tethered monotosylated DPEN ligand is selected from the group consisting of (R,R) or (S,S) N-(toluenesulfonyl),N-(propyl-3-phenyl)-1,2-diphenylenediamine (TsDPEN-teth), (R,R) or (S,S) N-(methanesulfonyl),N-(propyl-3-phenyl)-1,2-diphenylenediamine (MsDPEN-teth).

5. The process according to any one of the preceding claims, wherein the catalyst complex is selected from the group consisting of
[(R,R)-Ms-DPEN-teth RuCl] having the structural formula 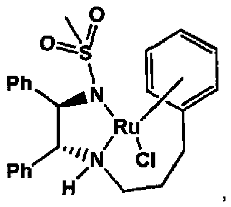 [(R,R)-Ts-DPEN-teth RuCl] having the structural formula 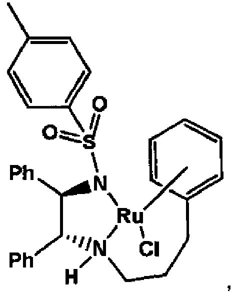 [(S,S)-Ms-DPEN-teth RuCl] having the structural formula 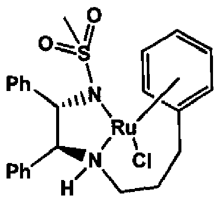 and [(S,S)-TsDPEN-teth RuCl] having the structural formula

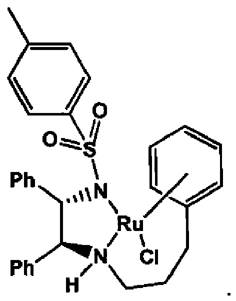

6. The process according to any one of the preceding claims, **characterized by** either one or a combination of the following procedural features (f) to (i):
(f) the transfer hydrogenation is carried out in a mixture comprising formic acid and triethylamine, preferably said mixture comprises formic acid and triethylamine in a volume ratio of 2/1 to 3/1, preferably 4.5/2 to 5.5/2;
(g) the transfer hydrogenation reaction is carried out at a temperature between 25 to 90°C, preferably 30 to 80°C, more preferably 35 to 70°C, even more preferably 36 to 60°C, yet even more preferably 38 to 50°C, and in particular 40 to 45°C;
(h) the transfer hydrogenation reaction is carried out for a reaction time of 15 to 100 h, preferably 20 to 70 h;
(i) the molar ratio of substrate to catalyst is at least 20/1, preferably 30/1 to 500/1, more preferably 35/1 to 250/1, even more preferably 40/1 to 150/1, yet even more preferably 75/1 to 125/1, and in particular 90/1 to 110/1.

7. A process for preparing a compound of formula II 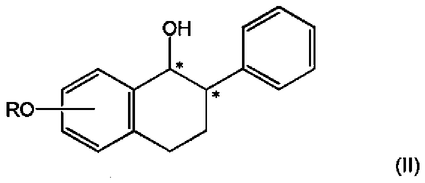 wherein -OR is an ether-type substituent,
by hydrogenation with elemental hydrogen (H₂) of a compound of formula I 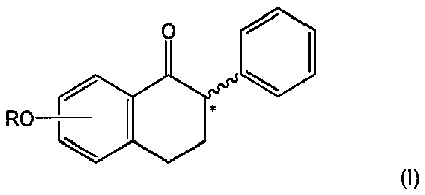 wherein -OR is defined as above, wherein hydrogenation is conducted in the presence of a catalyst selected from a complex comprising a transition metal.

8. The process according to claim 7, wherein -OR is a protecting group as defined in claim 1 or 2, and/or the catalyst is a catalyst as defined in claim 3.

9. The process according to claim 7 or 8, wherein the catalyst complex comprises a monotosylated DPEN ligand selected from the group consisting of (R,R) or (S,S) N-(methanesulfonyl)-1,2-diphenylenediamine (MsDPEN), (R,R) or (S,S) N-(toluenesulfonyl)-1,2-diphenylenediamine (TsDPEN), (R,R) or (S,S) N-(toluenesulfonyl),N•-(propyl-3-phenyl)-1,2-diphenylenediamine (TsDPEN-teth), (R,R) or (S,S) N-(methanesulfonyl),N-(propyl-3-phenyl)-1,2-diphenylenediamine (MsDPEN-teth).

10. The process according to any one of claims 7 to 9, wherein the catalyst complex is selected from the group consisting of
[(R,R)-Ms-DPEN RuCl (p-cym)] having the structural formula 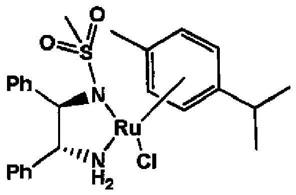 [(R,R)-Ts-DPEN RuCl (p-cym)] having the structural formula 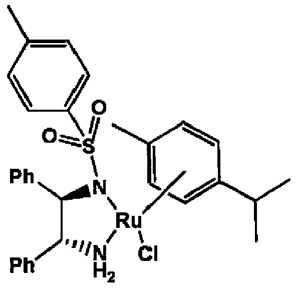 [(R,R)-Ms-DPEN-teth RuCl] having the structural formula 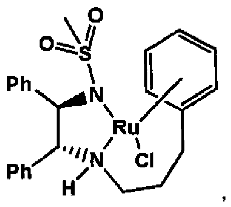 [(R,R)-Ts-DPEN-teth RuCl] having the structural formula 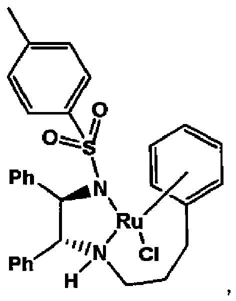 [(R,R)-MsDPEN RuCl (mes)] having the structural formula 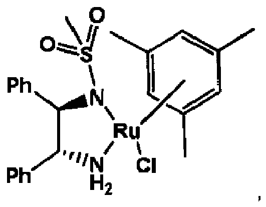 [(S,S)-Ms-DPEN RuCl (p-cym)] having the structural formula 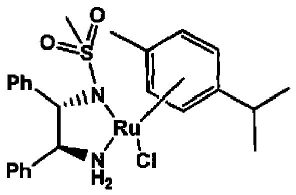 [(S,S)-Ts-DPEN RuCl (p-cym)] having the structural formula 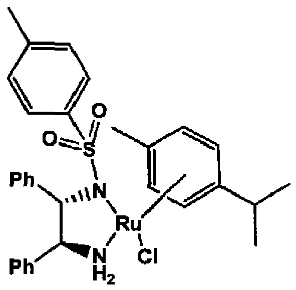 [(S,S)-Ms-DPEN-teth RuCl] having the structural formula 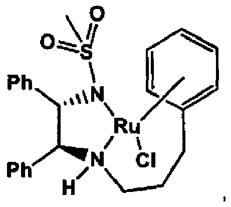 [(S,S)-Ts-DPEN-teth RuCl] having the structural formula 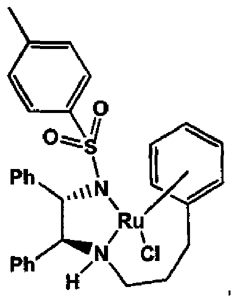 and
[(S,S)-Ms-DPEN RuCl (mes)] having the structural formula 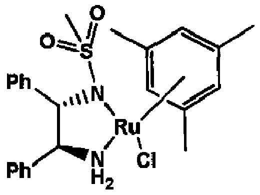 preferably the catalyst complex is selected from the group consisting of [(R,R)-Ms-DPEN-teth RuCl] and [(R,R)-Ms-DPEN RuCl (mes)], more preferably [(R,R)-MsDPEN RuCl (mes)].

11. The process according to any one of claims 7 to 10, **characterized by** either one or a combination of the following procedural features (i) to (vi):
(i) hydrogenation is carried out in a C1-C4 alcohol as the solvent, preferably methanol;
(ii) hydrogen is applied at a hydrogen pressure of 20 to 40 bar, preferably 25 to 35 bar, more preferable 28 to 32 bar;
(iii) hydrogenation reaction is carried out at a temperature between 30 to 80°C, preferably 35 to 75°C, more preferably 60 to 70°C;
(iv) hydrogenation reaction is carried out for a reaction time of 12 to 100 h, preferably 14 to 85 h, more preferably, in case reaction temperature is between 60 to 70°C, reaction time is 40 to 85 h;
(v) the molar ratio of substrate to catalyst is at least 20/1, preferably 30/1 to 500/1, more preferably 35/1 to 250/1, even more preferably 40/1 to 150/1, yet even more preferably 75/1 to 125/1, and in particular 90/1 to 110/1;
(vi) hydrogenation is carried out in the presence of an aqueous solution comprising an amine compound selected from the group consisting of triethylamine (Et₃N), diisopropyl ethyl amine (iPr₂EtN) and tetramethylguanidine (TMG); preferably, said aqueous solution comprises the amine compound in an amount of 10 to 40% by weight relative to the total weight of the aqueous solution.

12. The process according to any one of the preceding claims, wherein compound of formula II is obtained in a diastereomeric ratio of *cis*-isomer to *trans*-isomer of at least 100/1 and an enantiomeric excess of the *cis*-configured compound of at least 90%, more preferably enantiomeric excess of the *cis*-configured compound is at least 94%, even more preferably at least 96%, yet even more preferably at least 98%, and in particular at least 99%.

13. A process for preparing a compound of formula X 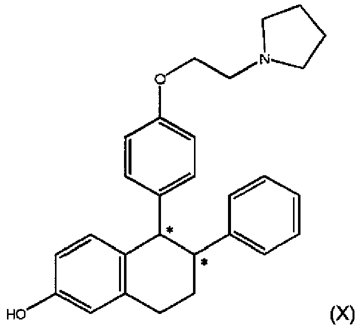 or a pharmaceutically acceptable salt thereof, the process comprising:
a) preparing a compound of formula II 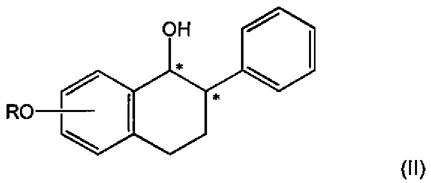 wherein -OR is an ether-type substituent,
by a process according to any one of claims 1 to 12, and
b) further converting the compound of formula II to compound of formula X or a pharmaceutically acceptable salt thereof.

14. A process according to claim 13, wherein compound of formula X is lasofoxifene (X') having the structural formula 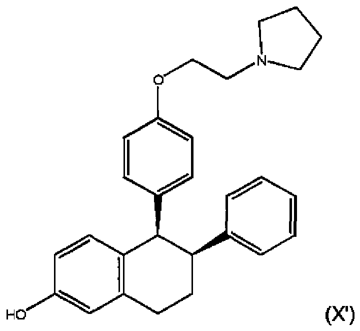 in which the substituents at 1- and 2-position are in (1*R,*2*S*)-configuration.

15. A process for the preparation of a pharmaceutical composition comprising a compound of formula X or a pharmaceutically acceptable salt thereof as active ingredient, comprising the steps of:
a) preparing compound of formula X or a salt thereof according to the process according to claim 13 or 14, and
b) admixing the prepared compound of formula X or pharmaceutically acceptable salt thereof with at least one pharmaceutically acceptable excipient.
